# EUROPÄISCHE PATENTSCHRIFT

(11) **EP 1 972 209 B1**
(45) Veröffentlichungstag und Bekanntmachung des Hinweises auf die Patenterteilung: **07.04.2010**
(21) Anmeldenummer: 08004048.8
(22) Anmeldetag: 05.03.2008
(51) Int. Cl.: A23L 2/52, A23L 2/68, A23L 1/30

(54) **Getränk**
Drink
Boisson

(30) Priorität: 20.03.2007 DE 102007013903
(43) Veröffentlichungstag der Anmeldung: 24.09.2008
(73) Patentinhaber: Coy, Johannes F., Dr., 64853 Otzberg (DE)
(72) Erfinder: Coy, Johannes F., 64853 Otzberg (DE)
(74) Vertreter: Rudolph, Ulrike

(56) Entgegenhaltungen:
- WO-A-2004/026294
- WO-A-2006/094716

## Beschreibung

Glukose spielt für den Menschen eine sehr wichtige Rolle bei verschiedensten Stoffwechselvorgängen. Viele Zellen des Menschen nutzen diesen Zucker als Energielieferant. Ein Überangebot von Glukose kann aber aufgrund der chemischen Eigenschaften von Glukose zu gravierenden Zellschäden und damit verbunden zu schweren Erkrankungen führen. Glukose besitzt die negative Eigenschaft, dass ein bestimmter Anteil des Moleküls in einer reaktiven Form (der offenen Aldehydform) vorliegt. Ähnlich wie beim Formaldehyd kommt es dann zu irreversiblen Reaktionen von Glukose mit Proteinen, die damit auf Dauer geschädigt werden. Dies lässt verstehen, dass eine zu hohe Konzentration von Glukose in der Zelle auf Dauer zu schweren Schäden führt. Manche Gewebe und Zellen des menschlichen Körpers sind besonders von zu hohen Glukosekonzentrationen betroffen. Dies sind genau die Gewebe, die bei Diabetikern auf Dauer durch hohe Glukosekonzentrationen geschädigt werden: Die Netzhaut (Retina), Nervenzellen (Neuronen) und Blutgefäßzellen (Endothelzellen). Über längere Zeit entwickeln sich chronische Diabeteslangzeitschäden wie Retinopathie, Neuropathie und Blutgefäßschäden, die dann schließlich zu Blindheit, Nervenschäden und Herzinfarkt führen können. Neben der schädigenden Wirkung von Glukose stellt sie den Treibstoff für Skelettmuskeln, aber auch den Treibstoff für das Aggressivwerden von Krebszellen dar.

Studien der jüngeren Zeit haben gezeigt, dass bestimmte Formen von Krebszellen Glukose als Treibstoff benötigen. Diese Form von Krebszellen ist äußerst aggressiv und nutzt das TKTL1-Enzym, um aus Glukose Energie zu gewinnen, auch wenn kein Sauerstoff zur Verfügung steht. Hierbei wird Glukose in Abwesenheit, aber auch in Anwesenheit von Sauerstoff zu Milchsäure (Laktat) vergoren. Glukose trägt damit zum Aggressivwerden von vorhandenen Krebszellen entscheidend bei. Obwohl solche vergärenden (fermentierenden) Krebszellen äußerst aggressiv sind und Metastasen bilden können, haben diese Krebszellen eine Achillesferse. Sie sind abhängig von Glukose. In vielen Fällen können diese Krebszellen nicht auf eine Fettverbrennung umstellen, weil die Mitochondrien abgeschaltet sind oder gar funktionsuntüchtig geworden sind. Diese Krebszellen sind damit absolut abhängig vom alleinigen Treibstoff Glukose. Krebs in der Form von lokal begrenzt wachsenden Tumoren kommt im ganzen Tierreich vor. Krebs in seiner aggressivsten Form, also metastasierender Krebs, kommt interessanterweise hauptsächlich nur beim Menschen mit westlicher Lebensweise und einigen von ihm gefütterten Haustieren als eine der Haupttodesursachen vor. Bei allen anderen Lebewesen stellt Krebs kein entscheidendes Problem dar. Der Tod durch Krebs beschränkt sich im Wesentlichen auf wenige Lebewesen: den Menschen mit westlicher Lebensweise, den Hund, die Hauskatze und im Labor gehaltene Tiere, die der Mensch füttert z.B. (Labormaus, Laborratte). Das heißt, die einzigen Lebewesen, die einen hohen Konsum von Kohlenhydraten aufweisen, welche bei der Verdauung schnell und viel Glukose freisetzen.

Wird bei der Verdauung von Lebensmitteln auf Dauer zu schnell und zu viel Glukose frei, so trägt dies entscheidend zum Entstehen von Krankheiten bei. Glukose führt hierbei zu einer Zellschädigung durch "advanced glycation endproducts" (AGE) und Radikalen. Dies trägt wesentlich zu der Entstehung von chronischen Diabetes-Langzeitschäden (Retinophathie, Neuropathie, Nephropathie und mikro- und makrovaskulären Schäden) bei. Hierdurch werden Krankheiten wie neurodegenerative Erkrankungen (z.B. Alzheimer, Wemicke-Korsakoff Syndrom, Demenz), Endothelzell- und Gefäßschäden (Herzinfarkt, Hirnschlag) und entzündliche Erkrankungen (Multiple Sklerose, Morbus Crohn, Ulzerative Kollitis, Rheuma) ausgelöst.

Eine Strategie, wie solche oben genannten Krankheiten, die durch zu hohe Glukosekonzentrationen ausgelöst werden, verhindert werden können, besteht in der Herstellung von flüssigen Lebensmitteln, die bei der Verdauung nur noch wenig und langsam Glukose freisetzen. Da Glukose außerdem auch den Treibstoff für vergärende Krebszellen darstellt, kann durch flüssige Lebensmittel, die bei der Verdauung nur noch wenig und langsam Glukose freisetzen., die Überlebenschance bei einer Krebserkrankung verbessert werden, indem eine Umstellung der Ernährung auf solche Nahrungsmittel, die nur wenig und langsam Glukose freisetzen, durchgeführt wird. Durch Zusatz von Milchsäure kann zudem durch Gabe des Endprodukts der Vergärung eine hemmende Wirkung auf den Vergärungsstoffwechsel in Krebszellen durch eine Endprodukthemmung (Milchsäure / Laktat) erreicht werden. Ein solches Lebensmittel in flüssiger Form kann hierbei als alleiniges Lebensmittel oder aber in Kombination mit anderen Lebensmitteln, die nur wenig und langsam Glukose freisetzen, zur Ernährung von Menschen oder Säugetieren eingesetzt werden. Die Ernährung kann als Prävention, als therapieunterstützende Maßnahme oder als alleinige Therapie eingesetzt werden.

Aufgabe der vorliegenden Erfindung ist deshalb die Bereitstellung eines solchen Lebensmittels.

Eine Lösung dieser Aufgabe besteht in der Bereitstellung eines Getränks, das durch die Kombination der folgenden Merkmale gekennzeichnet ist:
- Es weist einen Gehalt an Fett und/oder Öl auf, der wenigstens 5% Gewicht /Volumen und höchstens 35% Gewicht/Volumen beträgt. Mit anderen Worten: der Fett- und/oder Ölgehalt beträgt 5% Gewicht/Volumen oder mehr und er beträgt 35% Gewicht/Volumen oder weniger (5% Gew./Vol. ≤ Fett/Öl-Gehalt ≤ 35%Gew./Vol.).
- Dieser Gehalt an Fett und/oder Öl weist maximal 30% gesättigte Fettsäuren und mindestens 20% ungesättigte Fettsäuren auf.
- Die ungesättigten Fettsäuren umfassen Omega-3-Fettsäuren und Omega-6-Fettsäuren, wobei das Verhältnis von Omega-6-Fettsäuren zu Omega-3-Fettsäuren nicht höher als 4:1, bevorzugt nicht höher als 2:1 ist.
- Der Gehalt an Fett und/oder Öl umfasst pflanzliche Öle.
- Es (das Getränk) weist einen Gehalt an Kohlenhydraten von maximal 1,7% Gewicht/Volumen auf.
- Der Gehalt an Fett und/oder Öl ist höher als der Gehalt an Kohlenhydraten.
- Es (das Getränk) weist einen Gehalt an Milchsäure auf.

Aus der WO-A-2004/026294 ist ein Getränk bekannt, das etwa 4,4 % Gewicht Fett und Öl, etwa 17% Gewicht Kohlehydrate und etwa 0,04 % Gewicht Milchsäure enthält. Der Kohlehydratanteil bei diesem Getränk ist etwa viermal so groß wie der Fettanteil. Von diesem bekannten Getränk unterscheidet sich das erfindungsgemäße Getränk vor allem dadurch, dass es einen höheren Mindestgehalt an Fetten und/oder Ölen aufweist, dass es einen deutlich geringeren Kohlehydratanteil aufweist, der nur etwa 10% Gewicht des Kohlehydratanteils des bekannten Getränks beträgt, dass sein Fett-/Ölanteil höher ist als sein Kohlenhydratanteil, und dass es einen etwa siebenfach größeren Gehalt an Milchsäure aufweist.

In der WO-A-2006/094716 wird eine generelle Nahrungszusammensetzung vorgeschlagen, die einen Fett-/Ölanteil von 62% Gewicht und einen Kohlenhydratanteil von 12% Gewicht aufweist, wobei der Fett-/Ölanteil nur zu 15,6% Gewicht aus Omega-3- und Omega-6-Fettsäuren besteht. Ein Gehalt an Milchsäure ist nicht vorgeschlagen. Von dieser bekannten Nahrungszusammensetzung unterscheidet sich das erfindungsgemäße Getränk vor allem dadurch, dass sein Maximalgehalt an Fetten und/oder Ölen nur etwa die Hälfte desjenigen der bekannten Nahrungszusammensetzung beträgt, aber (etwa 30%) mehr ungesättigte Fettsäuren umfasst als dieser, dass es einen deutlich geringeren Kohlehydratanteil aufweist, der nur etwa 14% des Kohlehydratanteils der bekannten Nahrungszusammensetzung beträgt, und dass es einen Gehalt an Milchsäure aufweist.

Mit dem erfindungsgemäßen Getränk gehen vor allem die folgenden Vorteile einher:
Aufgrund des Verhältnisses höherer Gehalt an Fett und/oder Öl gegenüber niedrigerem Gehalt an Kohlenhydraten - vorzugsweise ist der Fett-/Ölanteil etwa zweimal so hoch wie der Kohlenhydratanteil - dient das Getränk als Energielieferant bei gleichzeitig niedriger Kohlenhydratzufuhr.
Aufgrund seines verhältnismäßig hohen Gehalts an essentiellen Fettsäuren dient das Getränk dem Konsumenten als bedeutende Quelle für lebensnotwendige und vom Körper selbst nicht herstellbare Biosynthesebausteine.
Aufgrund des Gehalts an Omega-6-Fettsäuren und Omega-3-Fettsäuren in dem genannten Mengenverhältnis wird im Konsumenten die Entstehung von entzündlichen Prozessen, bei der Arachidonsäure, Prostaglandine und Omega-6-Fettsäuren eine wichtige Rolle spielen, nicht gefördert oder gar gehemmt. Deshalb ist dieses Getränk besonders für Konsumenten bzw. Patienten mit entzündlichen Erkrankungen (Ulzerative Kolitis, Rheuma, Multiple Sklerose), für Patienten mit Diabetes und den damit einhergehenden chronischen Diabeteskomplikationen (Retinopahtien, Neuropathien, Nephropathien, mikro- und makrovaskulären Schäden z.B. Blutgefäßschäden, Herzinfarkt), für Patienten mit neurodegenerativen Erkrankungen (Alzheimer, Wernicke-Korsakoff Syndrom), und für Patienten mit Krebserkrankungen geeignet.
Aufgrund des Milchsäuregehalts wird beim Konsumenten der Vergärungsstoffwechsel von Glukose zu Laktat gehemmt (biochemisch als Endprodukthemmung bezeichnet). Zudem kann die enthaltene Milchsäure nicht über den TKTL1-Stoffwechselweg zur Energieproduktion herangezogen werden, da Milchsäure kein geeignetes Substrat für diesen Stoffwechsel ist. Deshalb empfiehlt sich dieses Getränk besonders für Konsumenten bzw. Patienten mit solchen Krebserkrankungen, bei denen der TKTL1-Glukosestoffwechsel aktiviert ist.
Der Milchsäuregehalt des Getränks hat außerdem den Vorteil, dass die Milchsäure im Darm des Konsumenten eine positive Wirkung auf die Darmflora entfaltet, unter anderem indem sie als Basenbildner die Entsäuerung unterstützt und als Energiesubstrat für Darmepithelien, Herz, Leber und Niere dient.

Der Gehalt an Milchsäure sollte wenigstens 0,3% Gewicht/Volumen betragen, Vorzugsweise beträgt er 1 bis 2 % Gewicht/Volumen, besonders bevorzugt sind 1,4% Gewicht/Volumen. Diese Gehaltswerte gewährleisten, dass einerseits die positiven Eigenschaften der Milchsäure merklich zum tragen kommen, und dass anderseits der Geschmack des Getränks nicht nachteilig beeinflusst wird.

Die Milchsäure kann in den beiden Formen L und D (rechtsdrehende L-(+)-Milchsäure und linksdrehende D-(-)-Milchsäure) vorliegen, damit sowohl die Bildung von L-Milchsäure über den Embden-Meyerhof-Weg und den Pentosephosphat-Weg als auch die Bildung von D-Milchsäure über den Methylglyoxalweg gehemmt wird. Bevorzugt ist hierbei ein höherer Gehalt an rechtsdrehender L-(+)-Milchsäure bis zu einem annähernd gleichen Volumen-Verhältnis von D- und L-Milchsäure zueinander.

Der Fett/Öl-Gehalt in dem Getränk besteht vorzugsweise ausschließlich aus pflanzlichen Fetten und/oder Ölen oder aus einer Mischung aus pflanzlichen und tierischen Fetten und/oder Ölen. In jedem Fall sollten die pflanzlichen Öle reich an ungesättigten Fettsäuren sein, weil ungesättigte Fettsäuren lebensnotwendig (essentiell) sind, und weil das Verhältnis der ungesättigten Fettsäuren Omega-3- zu Omega-6-Fettsäuren entscheidend dafür ist, ob eine Zelle oder ein Organismus gesund bleibt oder erkrankt.

Neben der Anwendung bei oder im Hinblick auf Krebs ist das erfindungsgemäße Getränk in allen genannten Varianten auch dazu geeignet und vorgesehen, die mit hohen Glukosekonzentrationen verbundenen Zellschädigungen, welche z.B. zu neurodegenerativen Erkrankungen, Diabeteslangzeitschäden, und mikro- und makrovaskulären Schäden wesentlich beitragen, deutlich zu reduzieren oder gar zu verhindern.

Das Getränk kann deshalb sowohl alkoholhaltig als auch nicht-alkoholhaltig sein.

Insbesondere für Sportler wird eine isotonische Variante des Getränks vorgeschlagen.

Das Getränk kann auch (noch) einen zusätzlichen Gehalt an Protein (Eiweiß) oder freien Aminosäuren aufweisen. Der Begriff 'freie Aminosäuren' bezeichnet hier Aminosäuren, die nicht in Peptidform (Di-, Tri-, Oligo-, Polypetid) sondern einzeln vorliegen. Der Protein- oder Aminosäuregehalt kann hierbei in einem Bereich von 0,1% bis 25% (Gewicht/Volumen; Gewicht/Gewicht), bevorzugt in einem Bereich von 1%-8% (Gewicht/Volumen; Gewicht/Gewicht) und ganz bevorzugt in einem Bereich von 1,5%-2,5% (Gewicht/Volumen; Gewicht/Gewicht) liegen. Bei den zugesetzten Proteinen (Eiweißen) handelt es sich vorzugsweise um solche, die zu einem hohen Prozentsatz aus ketogenen Aminosäuren aufgebaut sind und nur einen geringen Prozentsatz an glucogenen (glucoplastischen) Aminosäuren aufweisen, welche beim Abbau ihres Kohlenstoffgerüstes Glukose-Vorstufen wie Pyruvat, β-Ketoglutarat, Succinyl-CoA, Fumarat oder Oxalacetat freisetzen.
Bei den ketogenen Aminosäuren handelt es sich vorzugsweise um Threonin, Phenylalanin, Tyrosin, Tryptophan, Isoleucin, Lysin, und Leucin, ganz bevorzugt um die Aminosäuren Lysin und Leucin.

Des Weiteren können dem Getränk auch "freie" Aminosäuren zugesetzt werden. Zu diesem Zweck werden insbesondere die ketogenen Aminosäuren Threonin, Phenylalanin, Tyrosin, Tryptophan, Isoleucin, Lysin, und Leucin vorgeschlagen, wobei die Aminosäuren Lysin und Leucin besonders bevorzugt sind.
Durch den Einsatz von freien Aminosäuren kann der Anteil der ketogenen Aminosäuren gezielt erhöht werden, so dass die Glukoneogenese (also die Glukosebildung in der Leber aus Aminosäuren) beschränkt werden kann.

Der pH-Wert des Getränks sollte kleiner oder gleich pH 4,5 betragen. Damit geht der Vorteil einher, dass die Stabilität und Haltbarkeit des Getränks erhöht wird und dass es einen leicht säuerlichen Geschmack erhält, der vom Konsumenten als angenehm erfrischend empfunden wird.

Das erfindungsgemäße Getränk wird im folgenden anhand von Ausführungsbeispielen näher erläutert.

### Beispiel 1: Getränk-Variante 1

### Zutaten:

Joghurtkultur, Milch, Verdickungsmittel, Pflanzenöl 1 (z.B. Rapsöl), Pflanzenöl 2 (z.B. Traubenkernöl oder Hanföl oder Leinöl), Wasser;

### Herstellung:

Die Zutaten werden miteinander vermischt. Diese Mischung wird einem Gärungsprozess unterworfen. Nach Beendigung des Gärungsprozesses wird der pH-Wert eingestellt und damit ist die Getränkemischung bereit für eine Abfüllung in die Endgefäße.

### Beispiel 2: Getränk-Variante 2

### Zutaten:

Joghurtkultur, Sojabase, Verdickungsmittel, Zucker, Pflanzenöl 1 (z.B. Rapsöl oder Walnußöl), Pflanzenöl 2 (z.B. Traubenkernöl oder Hanföl oder Leinöl), Wasser;

### Herstellung:

Die Zutaten werden miteinander vermischt. Diese Mischung wird einem Gärungsprozess unterworfen. Nach Beendigung des Gärungsprozesses wird der pH-Wert eingestellt und damit ist die Getränkemischung bereit für eine Abfüllung in die Endgefäße

### Beispiel 3: Getränk-Variante 3

### Zutaten:

Sojabase, Verdickungsmittel, Pflanzenöl 1 (z.B. Rapsöl oder Hanföl), Pflanzenöl 2 (z.B.Traubenkernöl, oder Leinöl), Wasser, Milchsäure.

### Herstellung:

Die Zutaten werden miteinander vermischt. Der pH-Wert der Mischung wird eingestellt. Die Getränkemischung ist bereit für eine Abfüllung in die Endgefäße.
Im Unterschied zu den Getränke-Varianten 1 und 2 wird dieses Getränk während seiner Herstellung nicht fermentiert sondern ihm wird fertige Milchsäure zugesetzt.

## Patentansprüche

1. Getränk, **dadurch gekennzeichnet,**
- **dass** es einen Gehalt an Fett und/oder Öl von mindestens 5% Gewicht /Volumen und höchstens 35% Gewicht/Volumen aufweist,
- - **dass** dieser Gehalt an Fett und/oder Öl maximal 30% gesättigte Fettsäuren und mindestens 20% ungesättigte Fettsäuren aufweist, die Omega-3-Fettsäuren und Omega-6-Fettsäuren umfassen, wobei das Verhältnis von Omega-6-Fettsäuren zu Omega-3-Fettsäuren nicht höher als 4:1, bevorzugt nicht höher als 2:1 ist,
- - und **dass** dieser Gehalt an Fett und/oder Öl pflanzliche Öle umfasst,
- **dass** es einen Gehalt an Kohlenhydraten von maximal 1,7% Gewicht/Volumen aufweist,
- **dass** der Gehalt an Fett und/oder Öl höher ist als der Gehalt an Kohlenhydraten,
- und **dass** es einen Gehalt an Milchsäure aufweist.

2. Getränk nach Anspruch 1, **dadurch gekennzeichnet, dass** der Gehalt an Milchsäure wenigstens 0,3 % Gewicht/Volumen beträgt.

3. Getränk nach Anspruch 2, **dadurch gekennzeichnet**, das der Gehalt an Milchsäure 1 bis 2 % Gewicht/Volumen, vorzugsweise 1,4 % Gewicht/Volumen beträgt.

4. Getränk nach einem der Ansprüche 1 bis 3, **dadurch gekennzeichnet, dass** die Milchsäure in der D- und der L-Form (links- und rechtsdrehend) vorliegt.

5. Getränk nach Anspruch 4, **dadurch gekennzeichnet, dass** die L(+)-Milchsäure in einer höheren Konzentration vorliegt als die D(-)-Milchsäure.

6. Getränk nach einem der Ansprüche 1 bis 5, **dadurch gekennzeichnet, dass** der Gehalt an Fett und/oder Öl ausschließlich aus pflanzlichen Fetten und/oder Ölen besteht.

7. Getränk nach einem der Ansprüche 1 bis 5, **dadurch gekennzeichnet, dass** der Gehalt an Fett/Öl aus einer Mischung aus pflanzlichen und tierischen Fetten/Ölen besteht.

8. Getränk nach einem der Ansprüche 1 bis 7, **dadurch gekennzeichnet, dass** es nicht alkoholisch ist.

9. Getränk nach einem der Ansprüche 1 bis 7, **dadurch gekennzeichnet, dass** es alkoholisch ist.

10. Getränk nach einem der Ansprüche 1 bis 8, **dadurch gekennzeichnet, dass** es isotonisch ist.

11. Getränk nach einem der Ansprüche 1 bis 10, **dadurch gekennzeichnet, dass** es einen zusätzlichen Gehalt an Protein aufweist.

12. Getränk nach Anspruch 11, **dadurch gekennzeichnet, dass** der zusätzliche Gehalt an Protein zu einem Prozentsatz von 20 % bis 40 %, bevorzugt von 41 % bis 80 % und ganz bevorzugt von 81 % bis 100 % aus ketogenen Aminosäuren bestehen.

13. Getränk nach Anspruch 12, **dadurch gekennzeichnet, dass** die ketogenen Aminosäuren ausgewählt sind aus der Gruppe, bestehend aus: Threonin, Phenylalanin, Tyrosin, Tryptophan, Isoleucin, Lysin, und Leucin.

14. Getränk nach Anspruch 13, **dadurch gekennzeichnet, dass** es sich bei den ketogenen Aminosäuren um Lysin und/oder Leucin handelt.

15. Getränk nach einem der Ansprüche 1 bis 14, **dadurch gekennzeichnet, dass** es einen zusätzlichen Gehalt an freien Aminosäuren aufweist.

16. Getränk nach Anspruch 15, **dadurch gekennzeichnet, dass** die freien Aminosäuren ausgewählt sind aus der Gruppe ketogener Aminosäuren, bestehend aus: Threonin, Phenylalanin, Tyrosin, Tryptophan, Isoleucin, Lysin, und Leucin.

17. Getränk nach Anspruch 16, **dadurch gekennzeichnet, dass** es sich bei den freien ketogenen Aminosäuren um Lysin und/oder Leucin handelt.

18. Getränk nach einem der Ansprüche 1 bis 17, **dadurch gekennzeichnet, dass** es einen pH-Wert von kleiner oder gleich pH 4,5 aufweist.

## Claims

1. Beverage, **characterized in**
- **that** it contains a fat and/or oil content of at least 5% weight/volume and at most 35% weight/volume,
- - **that** this fat and/or oil content contains maximum 30% saturated acids and minimum 20% unsaturated fatty acids, which comprise omega-3-fatty acids and omega-6-fatty acids, where the ratio of omega-6 fatty acids to omega-3 fatty acids is no greater than 4:1, preferably no greater than 2:1
- - and **that** this content of fat and/or oil comprises vegetable oils,
- **that** it contains a carbohydrate content of maximum 1,7% weight/volume,
- **that** the content of fat and/or oil is higher than the carbohydrate content,
- and **that** it contains a content of lactic acid.

2. Beverage according to claim 1, **characterized in that** the content of lactic acid is at least 0,3 % weight/volume.

3. Beverage according to claim 2, **characterized in that** the lactic acid content amounts to 1% to 2% weight/volume, preferably 1,4% weight/volume.

4. Beverage according to one of claims 1 to 3, **characterized in that** the lactic acid is in the D and L forms (dextrorotatory and levorotatory).

5. Beverage according to claim 4, **characterized in that** the L-(+)-lactic acid is present in a higher concentration than the D-(-)-lactic acid.

6. Beverage according to one of claims 1 to 5, **characterized in that** the fat and/or oil content solely consists of vegetable fats and/or oils.

7. Beverage according to one of claims 1 to 5, **characterized in that** the fat/oil content consists of a mixture of vegetable and animal fats/oils.

8. Beverage according to one of claims 1 to 7, **characterized in that** it is not alcoholic.

9. Beverage according to one of claims 1 to 7, **characterized in that** it is alcoholic.

10. Beverage according to one of claims 1 to 8, **characterized in that** it is isotonic.

11. Beverage according to one of claims 1 to 10, **characterized in that** it additionally contains protein.

12. Beverage according to claim 11, **characterized in that** the added proteins comprise a percentage of 20% to 40%, preferably 41% to 80% and most preferably 81% to 100% ketogenic amino acids

13. Beverage according to claim 12, **characterized in that** the ketogenic amino acids are selected from the group consisting of threonine, phenylalanine, tyrosine, tryptophan, isoleucine, lysine and leucine.

14. Beverage according to claim 13, **characterized in that** the ketogenic amino acids are lysine and/or leucine.

15. Beverage according to one of claims 1 to 14, **characterized in that** it has an additional free amino acid content.

16. Beverage according to claim 15, **characterized in that** the free amino acids are selected from the group of ketogenic amino acids consisting of threonine, phenylalanine, tyrosine, tryptophan, isoleucine, lysine and leucine.

17. Beverage according to claim 16, **characterized in that** the free ketogenic amino acids are lysine and/or leucine.

18. Beverage according to one of claims 1 to 17, **characterized in that** its pH value is equal or less than pH 4,5.

## Revendications

1. Boisson, **caractérisée**
- **en ce qu'**elle présente une teneur en graisse et/ou en huile d'au moins 5 % en poids/volume et d'au plus 35 % en poids/volume,
- - **en ce que** cette teneur en graisse et/ou en huile comprend au plus 30 % d'acides gras saturés et au moins 20 % d'acides gras insaturés, qui comprennent des acides gras Oméga 3 et des acides gras Oméga 6, le rapport entre les acides gras Oméga 6 et les acides gras Oméga 3 n'étant pas supérieur à 4:1, de préférence pas supérieur à 2:1,
- - et **en ce que** cette teneur en graisse et/ou en huile comprend des huiles végétales,
- **en ce que** qu'elle comprend une teneur en glucides d'au plus 1,7 % en poids/volume,
- **en ce que** la teneur en graisse et/ou en huile est supérieure à la teneur en glucides,
- et en qu'elle comprend une teneur en acide lactique.

2. Boisson selon la revendication 1, **caractérisée en ce que** la teneur en acide lactique s'élève à au moins 0,3 % en poids/volume

3. Boisson selon la revendication 2, **caractérisée en ce que** la teneur en acide lactique est comprise entre 1 et 2 % en poids/volume, de préférence 1,4 % en poids/volume.

4. Boisson selon l'une des revendications 1 à 3, **caractérisée en ce que** l'acide lactique est présent sous la forme D et la forme L (lévogyre et dextrogyre).

5. Boisson selon la revendication 4, **caractérisée en ce que** l'acide L(+) lactique est présent dans une concentration plus importante que l'acide D(-) lactique.

6. Boisson selon l'une des revendications 1 à 5, **caractérisée en ce que** la teneur en graisse et/ou en huile se compose exclusivement de graisses et/ou d'huiles végétales.

7. Boisson selon l'une des revendications 1 à 5, **caractérisée en ce que** la teneur en graisse/huile se compose d'un mélange de graisses/huiles végétales et animales.

8. Boisson selon l'une des revendications 1 à 7, **caractérisée en ce qu'**elle n'est pas alcoolisée.

9. Boisson selon l'une des revendications 1 à 7, **caractérisée en ce qu'**elle est alcoolisée.

10. Boisson selon l'une des revendications 1 à 8, **caractérisée en ce qu'**elle est isotonique.

11. Boisson selon l'une des revendications 1 à 10, **caractérisée en ce qu'**elle présente en autre une teneur en protéine.

12. Boisson selon la revendication 11, **caractérisée en ce que** la teneur supplémentaire en protéine présente un taux d'acides aminés cétogènes de 20 % à 40 %, de préférence de 41 % à 80 % et de façon privilégiée de 81 % à 100 %.

13. Boisson selon la revendication 12, **caractérisée en ce que** les acides aminés cétogènes sont choisis dans le groupe composé de thréonine, phénylalanine, tyrosine, tryptophane, isoleucine, lysine et leucine.

14. Boisson selon la revendication 13, **caractérisée en ce que** les acides aminés cétogènes sont de la lysine et/ou de la leucine.

15. Boisson selon l'une des revendications 1 à 14, **caractérisée en ce qu'**elle présente en outre une teneur supplémentaire en acides aminés libres.

16. Boisson selon la revendication 15, **caractérisée en ce que** les acides aminés libres sont choisis dans le groupe des acides aminés cétogènes constitués de thréonine, phénylalanine, tyrosine, tryptophane, isoleucine, lysine et leucine.

17. Boisson selon la revendication 16, **caractérisée en ce que** les acides aminés libres sont la lysine et/ou la leucine.

18. Boisson selon l'une des revendications 1 à 17, **caractérisée en ce qu'**elle présente un pH inférieur ou égal à 4,5.
